(19) Europäisches Patentamt / European Patent Office / Office européen des brevets

(11) **EP 3 415 134 B1**

(12) **FASCICULE DE BREVET EUROPEEN**

(45) Date de publication et mention
de la délivrance du brevet:
**04.11.2020 Bulletin 2020/45**

(51) Int Cl.:
***A61G 7/057*** *(2006.01)*

(21) Numéro de dépôt: **18172721.5**

(22) Date de dépôt: **16.05.2018**

(54) **DISPOSITIF APTE À LA DETECTION, L'ANALYSE ET L'ALERTE POUR PREVENIR LA FORMATION DES ESCARRES**

DETEKTIONS-, ANALYSE- UND ALARMVORRICHTUNG ZUR VORBEUGUNG DER BILDUNG VON DEKUBITUS

DEVICE FOR DETECTION, ANALYSIS AND ALERT TO PREVENT ESCHAR FORMATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.06.2017 FR 1755311**

(43) Date de publication de la demande:
**19.12.2018 Bulletin 2018/51**

(73) Titulaire: **WINNCARE FRANCE**
**85670 St Paul Mont Penit (FR)**

(72) Inventeurs:
• **JOUET, Pascal**
**30000 Nimes (FR)**

• **HEBRARD, Jocelyn**
**30000 Nimes (FR)**
• **BARTHELAT, Julien**
**30000 Nimes (FR)**
• **GEAY, Pierre**
**30000 Nimes (FR)**

(74) Mandataire: **Demulsant, Xavier**
**Dejade & Biset**
**35, rue de Châteaudun**
**75009 Paris (FR)**

(56) Documents cités:
**EP-A1- 2 508 128      US-A1- 2005 172 398**
**US-A1- 2009 270 770      US-A1- 2014 039 351**

**EP 3 415 134 B1**

## Description

L'invention a trait au domaine de la prévention de la formation d'escarres, et plus particulièrement aux dispositifs permettant, par le biais d'une nappe de capteurs de mouvements et d'un procédé d'analyse de ces mouvements, de prévenir la formation d'escarres.

L'invention trouve notamment application dans les lits, en particulier les lits médicalisés.

Par « escarres » on désigne ici les ulcérations ou nécroses cutanées qui font suite à une compression prolongée des parties molles sur une saillie osseuse sous-jacente, au niveau d'une surface d'appui du corps. Les escarres sont des lésions cutanées d'origine ischémiques.

Les facteurs de risque des escarres sont conventionnellement classés en facteurs extrinsèques ou mécaniques (en particulier pression, friction, cisaillement), et facteurs intrinsèques ou cliniques (en particulier immobilité, nutrition, incontinence, état de la peau, baisse du débit circulatoire, neuropathie, âge, antécédent d'escarres).

Les facteurs de risques d'apparition d'escarres sont multiples, et restent objet de recherches, voir par exemple Coleman et al, Patient risk factors for pressure ulcer development : systematic review, International journal of nursing studies (2013), pp.974-1003.

*L'European Pressure Ulcer Advisory Panel* (EPUAP) a élaboré des recommandations en matière de prévention des escarres, en liaison avec *l'American National Pressure Ulcer Advisory Panel* (NPUAP). Dans leur version de 2014, ces recommandations comprennent de très nombreux paramètres altération de l'intégrité cutanée (par exemple peau sèche, excès d'humidité, érythème, œdème, induration, chaleur locale), indicateurs nutritionnels (par exemple malnutrition, anémie, taux d'hémoglobine, albuminémie, poids), facteurs affectant la circulation et l'oxygénation (par exemple diabète, pression artérielle basse), pression, friction et cisaillements, fréquence et techniques de repositionnement, âge avancé, état de santé générale, température corporelle.

L'évaluation du risque d'escarres devrait ainsi prendre en compte un vaste ensemble de facteurs tels que l'oxygénation et la vascularisation des tissus, l'état de la peau, l'activité physique et la mobilité, l'état nutritionnel.

De très nombreuses échelles d'évaluation du risque ont été proposées: échelle de Norton, cf. Anthony et al, Norton, Waterlow and Braden scores : a review of the literature and a comparison between the scores and clinical judgment, Journal of Clinical Nursing (2008), pp. 646-653; échelle de Braden, cf. Chen et al, Braden scale for assessing ulcer risk in hospital patients : a validity and reliability study, Applied Nursing Research (2017), pp. 169-174*;* échelle de Waterloo, cf. Serpa et al, Validity of the Braden and Waterloo subscales in predicting pressure ulcer risk in hospitalized patients, Applied Nursing Research (2011), pp. e23-e28*;* échelle de Cubbin et Jackson, cf. Seongsook et al, Validity of pressure ulcer risk assessment, Cubbin and Jackson, Braden and Douglas scale, International Journal of Nursing Studies (2004), pp. 199-204*;* échelles francophones de Colin et Lemoine, échelle des Peupliers-Gonesse, échelle d'Angers, échelle de Genève, cf. Boisson, Optimisation de la prévention ambulatoire des ulcères cutanés dus à la pression, Thèse de docteur en pharmacie (2016*)*.

Les échelles d'évaluation du risque d'escarres présentent des limitations. En particulier, leur utilisation ne résulte pas d'une évaluation continue de la personne, et l'observateur induit une subjectivité inévitable. Il est fréquent que des personnes présentant des risques élevés, au vu de leur évaluation par ces échelles, ne développent pas d'escarres. Et il est également fréquent que des personnes présentant des risques faibles développent des escarres. Une présentation des limitations des échelles existantes est présentée par Torressan, Prévalence des escarres dans les établissements pour personnes âgées dépendantes en Aquitaine, Thèse, (2015*)*.

Malgré leurs limitations, les échelles d'évaluation des risques d'escarres sont conventionnellement prises en compte pour décider de la mise en place et de la prise en charge financière des moyens techniques de prévention des escarres. Par exemple, en France, pour déterminer le seuil de prise en charge par l'assurance maladie du coût des matelas, sur-matelas et coussins d'aide à la prévention des escarres, l'échelle de Norton est utilisée, un critère de prise en charge étant score inférieur ou égal à 14 sur l'échelle de Norton ou un patient ayant un risque équivalent, évalué par une autre échelle validée.

L'apparition d'une escarre entraîne souvent une baisse importante de la qualité et du confort de vie, ainsi qu'une gêne et une souffrance physique, notamment lors des soins.

L'impact des escarres en termes de santé publique est très élevé. Une évaluation des coûts de prévention et traitement des escarres est présentée par Démarré et al, The cost of prevention and treatment of pressure ulcers : a systematic review, International journal of nursing studies (2015), pp. 1754-1774*.*

Les escarres sont vues comme des événements indésirables lors d'une hospitalisation, pouvant conduire à des recours en justice, fréquents aux Etats Unis.

Les moyens techniques mis en œuvre pour la prévention des escarres sont typiquement des coussins en gel, des coussins en mousse à mémoire de forme, des matelas en mousse mono-densité en forme de gaufrier, des sur-matelas en mousse viscoélastique à mémoire de forme, des sur-matelas à air (à pression constante, dynamique, ou mixte ou à perte d'air), des matelas comprenant des inserts dans les zones à risque d'escarre (insert à eau).

Les zones à risque et localisations préférentielles des escarres sont le sacrum et les talons, en particulier chez les patients alités en position dorsale. D'autres localisations sont possibles, comme les ischions, le trochanter (chez les personnes alitées en position latérale), ou l'occiput, tous les points d'appui pouvant présenter une escarre, par exemple les coudes ou les genoux.

La position dans laquelle le patient se trouve le plus souvent a un impact sur la localisation la plus probable d'une escarre. Ainsi, par exemple, à un décubitus dorsal sont associées des escarres à l'occiput, au sacrum, aux talons, au rachis dorsal, aux omoplates et aux coudes.

Pour identifier les zones à pression élevée, on connaît des nappes de capteurs résistifs ou capacitifs, fournissant une carte avec zones de couleurs de même index de pression (*pressure area index*). Le document EP1664712 décrit l'utilisation d'un dispositif de mesure de pression d'interface dans un dispositif anti-escarre, un tel capteur de pression d'interface étant proposé en intégration dans un siège, cf. Meffre et al Mapi : active interface pressure sensor intergrated into a seat, IRBM (2008), pp. 375-379 (voir aussi Meffre, Conception et réalisation d'une instrumentation dédiée à la prédiction du confort d'assise et à la prévention des escarres, Thèse INSA Lyon, 2007).

L'emploi d'algorithmes de traitement de données a été proposé dans la littérature, afin de fournir des niveaux de risques d'escarres, ces algorithmes traitant des données issues d'une surveillance continue et automatique des patients. Un état des recherches pour de telles méthodes peut être trouvé dans le document Marchione et al Approaches that use software to support the prevention of pressure ulcer : as systematic review, International Journal of Medical Informatics (2015), pp. 725-736. Sur les 36 articles scientifiques identifiés par Marchione et al, 26 décrivent l'emploi de capteurs de la pression exercée sur le matelas par le corps du patient et 11 proposent de déduire la position du patient du relevé des pressions exercées sur le matelas.

Les méthodes proposées dans les travaux de recherche identifiés par Marchione et al présentent au moins un ou plusieurs des inconvénients suivants :

- aucune alerte d'un risque d'escarre n'est délivrée ;
- l'impact des moyens de contrôle sur le confort du patient n'est pas pris en compte ;
- l'impact des moyens de contrôle sur l'hygiène n'est pas pris en compte ;
- l'efficacité de la méthode dans la prévention des escarres n'est pas démontrée, et aucune comparaison n'est effectuée avec un groupe témoin.

Le document JPH 11-342161 (Denso) décrit un dispositif d'enregistrement des postures d'un individu alité, par le biais de capteurs de pression disposés entre le sommier et le matelas d'un lit. Le dispositif est relié à un boîtier apte analyser les positions de l'individu et à afficher le temps d'appui sur le matelas de chacun des membres de l'individu. Cet affichage est configuré pour afficher une alerte, lorsque le temps d'appui d'un des membres dépasse un temps prédéfini.

Le document JPH 8-238275 (Yokohama Imeeji System) décrit un dispositif de mesure du temps d'appui du corps de l'individu sur un caoutchouc conducteur sensible à la pression. Le dispositif comprend également un boîtier configuré pour effectuer une alerte, lorsque le temps d'appui est supérieur à un temps prédéfini.

Le document Panfil et al Häufigkeit von Bewegung im Bett zur Einschätzung der Dekubitusgefahrdung - Eine systematisierte Übersichtsarbeit, International journal of health professions (2014) pp. 61-72 après une recherche approfondie dans la littérature, conclu à l'absence de données probantes quant au lien entre la fréquence des mouvements d'un patient dans un lit et le risque d'escarres.

Le document EP2508128 se rapporte à la détection de changement de position de patients, par exemple sur un lit. Un processeur est configuré pour générer des alarmes en fonction de la position détectée du patient, par exemple une alarme est déclenchée lorsque le patient est sorti du lit, ou est assis au bord du lit. Des capteurs de pression peuvent détecter les variations de pression sur le matelas, le processeur pouvant adapter la pression dans le matelas gonflable. Des capteurs permettent de détecter les changements mineurs de position du patient, cette détection permettant de calculer un score de mobilité et un score d'activité, et ces scores sont présentés comme liés à des risques de santé, dont celui d'apparition d'escarres. Le document EP2508128 indique qu'un risque élevé d'escarre peut être associé ou non à un score de mobilité élevé, d'autres facteurs pouvant être pris en compte comme la position dans le lit, la fermeté du matelas, l'humidité, l'alimentation du patient, les frottements.

Le document US 2005/172398 décrit l'utilisation de capteurs de pression permettant d'augmenter ou diminuer la tension de bandes formant le support pour le poids du patient, sur une assise de fauteuil ou dans un lit. Un des objectifs est de déterminer s'il est nécessaire de déplacer le patient, en évitant un déplacement du patient qui le ramènerait vers la position qu'il vient de quitter.

Contrairement aux enseignements dominants l'état de la technique, mettant systématiquement en avant l'importance cruciale d'une pression appliquée durant un temps d'appui long, dans l'apparition d'escarres, les inventeurs ont considéré un paradigme très différent, selon lequel le risque d'apparition d'escarres est lié à la fréquence et l'amplitude des mouvements d'une personne, la fréquence de mouvements de grande amplitude pouvant s'avérer efficace dans la recirculation capillaire.

Un premier objectif est de proposer un procédé pour fournir un indicateur de risques d' escarres.

Un deuxième objectif est de proposer un tel procédé, mesurant la fréquence et l'amplitude des mouvements d'un individu alité.

Un troisième objectif est de proposer un tel procédé, analysant les mesures effectuées.

Un quatrième objectif est de proposer un tel procédé, délivrant une alerte, en fonction du résultat de l'analyse des mesures.

Un cinquième objectif est de proposer un dispositif apte à mettre en oeuvre un procédé répondant aux objectifs précédents.

A ces fins, il est proposé, en premier lieu, un procédé pour fournir un indicateur de risques d' escarres d'un individu reposant sur un support, le procédé comprenant :

- une étape de mesure d'au moins une grandeur, par le biais de capteurs disposés entre le support et l'individu ;
- une étape de calcul d'un indicateur d'une surface de contact de l'individu avec le support, à partir de ladite au moins une grandeur ;
- une étape de détection de présence de l'individu sur le support, en fonction de la surface de contact de l'individu avec le support ;
- une étape de détection d'absence de mouvement réalisé par l'individu, en fonction d'une variation dans le temps de l'indicateur de la surface de contact calculée, si, au cours de l'étape de détection de présence, la présence d'un individu est détectée sur le support ;
- une étape de détection d'amplitude des mouvements réalisés par l'individu, en fonction de la variation dans le temps de l'indicateur de la surface de contact calculée, si, au cours de l'étape de détection d'absence de mouvement, une absence de mouvement n'est pas détectée ;
- une étape de notification de l'état de l'individu.

La détection de l'amplitude des mouvements réalisés par la personne permet de distinguer des petits mouvements, des mouvements segmentaires sans impact sur la posture, et des mouvements de grande amplitude, notamment des retournements.

La détection de l'amplitude des mouvements permet en outre de mesurer la durée séparant deux mouvements de grande amplitude.

Diverses caractéristiques supplémentaires peuvent être prévues, seules ou en combinaison:

- l'étape de notification de l'état de l'individu comprend :

  ○ une étape d'information de l'état de l'individu, émettant des résultats de l'étape de détection de présence de l'individu, de l'étape de détection d'absence de mouvement et de l'étape de détection d'amplitude des mouvements, cette étape d'information étant émise régulièrement ;
  ○ une étape d'alerte de non-présence de l'individu sur le support, cette étape d'alerte de non-présence de l'individu étant émise si, à l'étape de détection de présence de l'individu, aucun individu n'est détecté pendant un temps prédéterminé ;
  ○ une étape d'alerte d'absence de mouvement de l'individu, cette étape d'alerte d'absence de mouvement étant émise si, à l'étape de détection d'absence de mouvement, aucun mouvement n'est détecté pendant un temps déterminé ;
  ○ une étape d'alerte d'amplitude des mouvements de l'individu, cette étape d'alerte d'amplitude des mouvements étant émise si, à l'étape de détection d'amplitude des mouvements, un petit mouvement est détecté pendant un temps prédéterminé ;

- l'étape de notification comprend une étape d'alerte d'agitation de l'individu, cette étape d'alerte d'agitation étant fonction d'un nombre prédéterminé de mouvements détectés pendant un temps donné ;
- ladite au moins une grandeur mesurée à l'étape de mesure est une grandeur électrique, et l'étape de calcul comprend :

  ○ une première étape de calcul d'un indicateur d'une capacité générée par les capteurs ;
  ○ une deuxième étape de calcul d'un indicateur d'une longueur active de chaque capteur ;
  ○ une troisième étape de calcul d'un indicateur d'une aire ;
  ○ une quatrième étape de calcul de l'indicateur de la surface de contact ;
  ○ une cinquième étape de calcul de la variation dans le temps de l'indicateur de la surface de contact.

Il est proposé, en deuxième lieu, un dispositif pour la prévention de la formation d'escarres, ce dispositif étant apte à réaliser le procédé tel que présenté ci-dessus, le dispositif comprenant :

- les capteurs disposés entre le support et l'individu, les capteurs étant disposés de sorte à indiquer la présence d'un objet sur le support ;
- un boîtier relié aux capteurs, le boîtier étant muni :

  ◦ d'un élément de contrôle, configuré pour réaliser les étapes du procédé ;
  ◦ d'une mémoire, apte à enregistrer les étapes du procédé ;

- un terminal de suivi, apte à afficher l'étape de notification de l'état de l'individu.

Diverses caractéristiques supplémentaires peuvent être prévues, seules ou en combinaison:

- les capteurs présentent chacun la forme d'une bande ;
- les capteurs sont sensiblement perpendiculaires à une direction suivant laquelle s'étend le corps de l'individu ;
- les capteurs sont des capteurs capacitifs ;
- le dispositif comprend un faisceau, reliant les capteurs et le boîtier, le faisceau comprenant plusieurs parties indépendantes ;
- le faisceau comprend deux ou trois parties.

D'autres caractéristiques et avantages de l'invention apparaîtront plus clairement et de manière concrète à la lecture de la description ci-après de modes de réalisation, laquelle est faite en référence aux dessins annexés dans lesquels :

- la figure 1 est une vue schématique d'un dispositif de détection, d'analyse et d'alerte, pour prévenir la formation des escarres ;
- la figure 2 est une représentation schématique, illustrant des étapes relatives à un mode de réalisation d'un procédé de mesure, d'analyse et d'alerte prévenant la formation des escarres ;
- la figure 3 est une représentation schématique d'une surface de contact du corps d'un individu reposant sur le dispositif, en position de décubitus dorsal ;
- la figure 4 est un graphique représentant la capacité de trois faisceaux dont les capteurs sont respectivement contrôlés par un, deux ou trois contrôleurs.

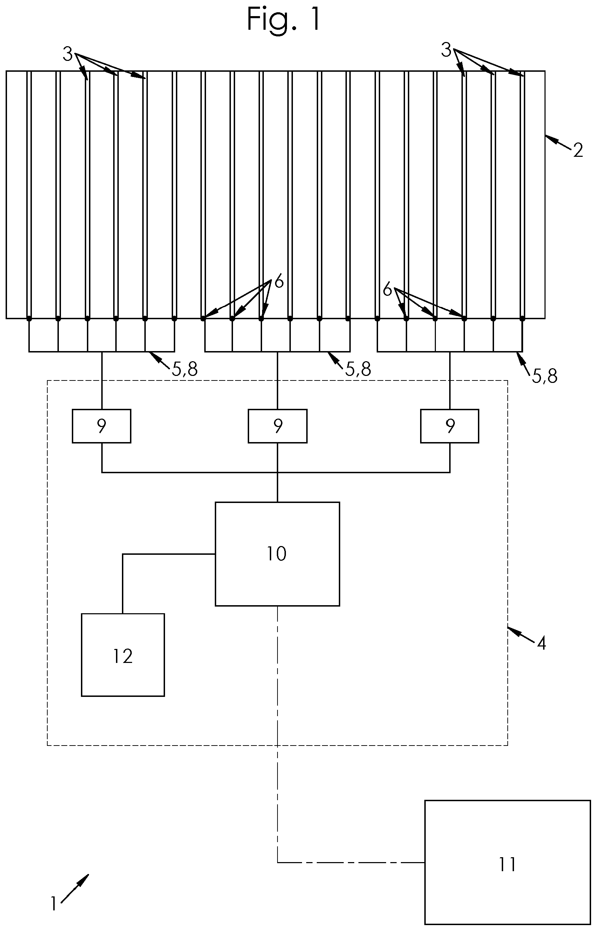

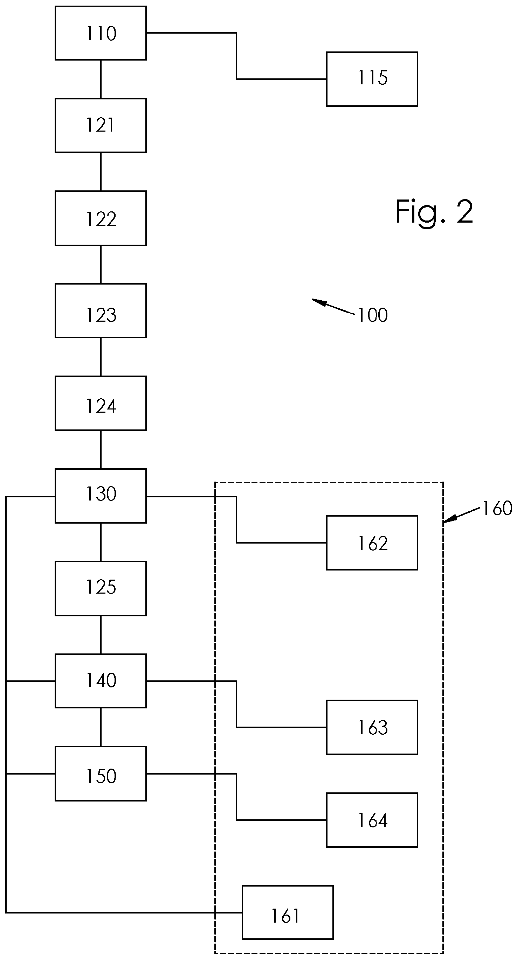

Sur la figure 1 est représenté un dispositif **1** pour la prévention de la formation d'escarres, le dispositif **1** comprenant une housse **2,** munie de capteurs **3** et une protection recouvrant les capteurs **3.**
Le dispositif **1** est apte à être posé sur le matelas d'un lit.
Le dispositif **1** comprend également un boîtier **4,** relié aux capteurs **3** par le biais d'un faisceau **5,** apte à mesurer et analyser des données fournies par les capteurs **3** et à émettre une alerte, en fonction du résultat de ladite analyse, conformément à un procédé **100** représenté sur la figure 2.
Le lit, le matelas et la protection ne sont pas représentés sur les figures, pour des raisons de simplicité.
Les inventeurs sont partis de l'hypothèse que la prévention des escarres serait améliorée par une fréquence et une amplitude importante des mouvements d'un individu reposant sur le lit.
Trois niveaux de mouvements sont pris en compte :

- une absence de mouvement,
- des petits mouvements, correspondant à une flexion inférieure à 30° d'une jambe de l'individu lorsque celui-ci est en position de décubitus latéral,
- et des grands mouvements, correspondant à une flexion supérieure à 30° d'une jambe de l'individu lorsque celui-ci est en position de décubitus latéral.

Dans une mise en oeuvre, une surface **R** de référence étant le quatre-vingt quinzième percentile de la surface **S** de contact d'un individu reposant sur le lit, pendant un temps prédéterminé :

- les petits mouvements sont définis comme une variation $\Delta$**S** de la surface **S** inférieure ou égale à 11 pourcents d'une surface **R** de référence, pendant une durée de 400 millisecondes ;
- les grands mouvements sont définis comme la variation $\Delta$**S** de la surface **S** supérieure à 11 pourcents de la surface **R** de référence, pendant une durée de 400 millisecondes.

La présence de l'individu sur le lit est définie par une surface S de contact supérieure à 20 pourcents de la surface **R** de référence, pendant une durée supérieure à 800 millisecondes.

Selon un mode de réalisation avantageux, la housse **2** enveloppe intégralement le matelas, par exemple un matelas mousse.

Avantageusement, la housse **2** est lavable. Le procédé et le dispositif selon l'invention n'ont ainsi aucun impact sur l'hygiène.

Selon des modes de réalisation, la housse **2** enveloppe partiellement le matelas ou la housse **2** est apte à être posée sur un support différent d'un lit, permettant ainsi le suivi d'un individu reposant sur ledit support.

Les capteurs **3** se présentent, de préférence, sous la forme de bandes. Avantageusement, les bandes sont disposées sensiblement parallèlement entre elles. Selon le mode de réalisation préféré, les capteurs **3** sont distants les uns des autres.

Les capteurs **3** sont de préférence disposés suivant une direction sensiblement perpendiculaire à une direction générale suivant laquelle s'étend le corps de l'individu. Selon le mode de réalisation illustré sur la figure 1, les capteurs **3** sont disposés dans la largeur d'un matelas.

Avantageusement, la housse **2** est suffisamment résistante pour ne pas être dégradée si une matière plus ou moins liquide se répand sur celle-ci résultant, par exemple, d'une incontinence de l'individu reposant sur la housse **2**, d'un lavage ou d'une stérilisation de celle-ci.

Chaque capteur **3** comprend un connecteur **6**, permettant une liaison entre le faisceau **5** et les capteurs **3** et leurs séparations, lors du déplacement, du lavage ou de la stérilisation de la housse **2**.

Les connecteurs **6** sont, de préférence, disposés à une extrémité des capteurs **3**, de sorte que les connecteurs **6** et le faisceau **5** ne gênent pas un individu reposant sur la housse **2**.

Selon un mode de réalisation, la housse **2** comprend dix-huit capteurs **3** de largeur **l** égales. Les capteurs **3** sont séparés deux à deux d'une même distance **d**.

Selon un mode de réalisation, la largeur **l** des capteurs **3** est de 10 millimètres. La distance **d** séparant les capteurs **3** est égale à 100 millimètres.

Avantageusement, les capteurs **3** sont fabriqués dans un matériau flexible. Selon un mode de réalisation, les capteurs **3** sont fabriqués en un tissu conducteur d'électricité.

Les capteurs **3** sont avantageusement disposés entre le matelas et la protection.

Les capteurs **3** sont avantageusement des capteurs **3** capacitifs. Ainsi, les capteurs **3** et le corps de l'individu disposé sur la housse **2** définissent deux armatures séparées un matériau isolant défini par la protection.

Lors d'une mesure, les capteurs 3 génèrent une capacité $C_v$ à vide, si aucun individu ne repose sur le matelas. Plus le corps de l'individu est disposé en regard d'un capteur **3** plus une capacité **C** générée est importante.

Il est ainsi possible d'établir une schématisation de la surface S de contact du corps de l'individu avec la protection, en effectuant une mesure par le biais de tous les capteurs **3**.

La surface **S** de contact est calculée comme la somme des aires $A_n$ de trapèzes **7**, dont les bases sont définies par une longueur **L** active de deux capteurs **3** voisins.

La longueur **L** active de chaque capteur **3** correspond à une portion dudit capteur **3** en contact avec le corps de l'individu.

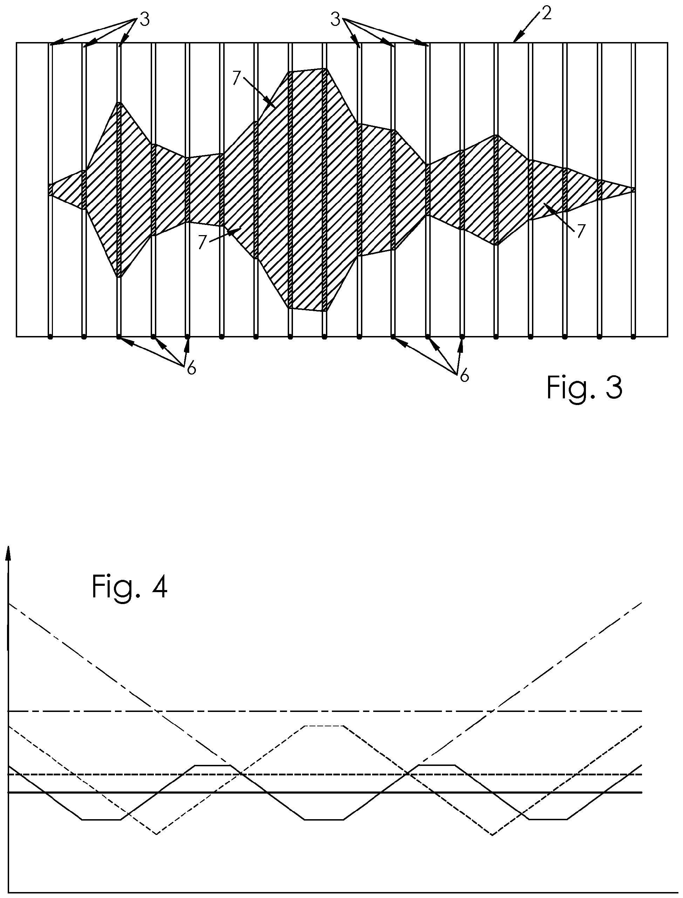

La figure 3 illustre un exemple d'une telle surface **S** de contact, pour un individu reposant sur le matelas en position de décubitus dorsal, la tête de l'individu étant positionnée sur la gauche de la figure et les pieds de l'individu étant positionnés sur la droite de la figure.

Dans le mode de réalisation décrit, la surface **S** ne prend pas en compte l'aire de chaque capteur en contact avec le corps de l'individu.

Selon des modes de réalisation différents, le calcul de la surface **S** prend en compte l'aire de chaque capteur en contact avec le corps de l'individu. Les aires $A_n$ sont calculées en fonction de la forme et de la disposition des capteurs **3**, les aires ne sont donc pas forcément des aires $A_n$ de trapèzes.

La capacité **C** générée est obtenue par l'ajout d'une capacité générée par le faisceau **5** reliant les capteurs **3** au boîtier **4** à une capacité générée par les capteurs **3**.

La capacité du faisceau **5** peut être considérée comme proportionnelle à sa longueur. Afin de minimiser les risques d'erreurs lors de la mesure de la capacité de chacun des capteurs **3**, il est donc avantageux de diminuer le plus possible la longueur du faisceau **5**.

Il s'est avéré, à la suite d'essais présentés dans le tableau **T** ci-dessous et sur la figure **4**, que la division du faisceau **5** en plusieurs parties **8** indépendantes permet de réduire la longueur totale du faisceau **5** et donc la capacité générée par le faisceau **5**.

Le tableau **T**, ci-après, présente la longueur du faisceau **5** reliant chaque capteur **3** au boîtier **4** et la capacité en résultant pour un faisceau **5** comprenant une partie **8**, deux parties **8** ou trois parties **8**.

Le tableau **T** présente également la valeur moyenne de la capacité générée par le faisceau, calculée pour chacun des faisceaux **5** présentés.

La figure **4** est un graphique représentant les valeurs du tableau **T**. Le faisceau **5** comprenant une seule partie **8** est représenté en traits mixtes, le faisceau **5** comprenant deux parties **8** est représenté en traits pointillés et le faisceau **5** comprenant trois parties **8** est représenté en traits pleins. Les valeurs moyennes de la capacité générée par les faisceaux **5** sont illustrées en trait gras.

Le tableau **T** et la figure 4 illustrent clairement que plus le faisceau **5** est divisé en un nombre important de parties **8,** plus la capacité générée par le faisceau **5** est faible.-

Selon le mode de réalisation représenté sur la figure **1**, le faisceau **5** du dispositif **1** comprend trois parties **8**. Selon des modes de réalisation différents, le faisceau **5** pourrait être divisé en un nombre différent de parties **8**.

Les parties **8** sont, de préférence, identiques.

Chaque partie **8** du faisceau **5** est reliée à un élément **9** de mesure appartenant au boîtier **4**.

Les éléments **9** de mesure sont configurés pour réaliser périodiquement des phases de charge et de décharge des capteurs **3**. Les phases de charge et de décharge sont réalisées à un courant i constant. Une tension **V** est mesurée, aux bornes de chaque capteur **3** par leur élément **9** de mesure respectif, à la fin de la phase de charge.

Les phases de charge et de décharge durent un temps **T** d'échantillonnage prédéfini. Les phases de charge et de décharge ont, de préférence, la même durée, c'est-à-dire la moitié du temps **T** d'échantillonnage.

Selon le mode de réalisation préféré, le temps **T** d'échantillonnage est de 1 milliseconde.

Les éléments **9** de mesure effectuent la mesure de la tension **V** sur requête d'un élément **10** de contrôle appartenant au boîtier **4** selon une étape **110** de mesure du procédé **100** représenté sur la figure 2.

L'élément **10** de contrôle calcul la capacité **C** générée par chacun des capteurs **3**, au cours d'une première étape **121** de calcul du procédé **100**, avec la formule suivante :

$$C = i \times \frac{T}{V}$$

Des résultats d'essais ont révélé que, pour assurer une bonne qualité de mesure, la tension **V** mesurée devait être comprise entre une tension **V<sub>min</sub>** minimale et une tension **V<sub>max</sub>** maximale telles que :

$$V_{min} = 0.7$$

$$V_{max} = VDD - 0.7$$

la variable **VDD** étant la tension d'alimentation de l'élément **9** de mesure.

La tension d'alimentation de l'élément **9** de mesure est, par exemple, de 3,3 volts ou 5,0 volts.

La tension **V<sub>min</sub>** minimale et la tension **V<sub>max</sub>** maximale permettent de définir une capacité **C<sub>min</sub>** minimale et une capacité **C<sub>max</sub>** maximale aptes à être générées par chaque capteur **3**.

La capacité **C<sub>min</sub>** minimale et la capacité **C<sub>max</sub>** maximale sont égales à :

$$C_{min} = i \times \frac{T}{V_{max}} \qquad\qquad C_{max} = i \times \frac{T}{V_{min}}$$

$$C_{min} = i \times \frac{T}{VDD - 0.7} \qquad\qquad C_{max} = i \times \frac{T}{0.7}$$

Avantageusement, l'élément **10** de contrôle réalise une moyenne des valeurs de la tension **V** mesurée au terme de plusieurs phases de charge consécutives ou une moyenne des valeurs de la capacité **C** générée, calculée au terme de plusieurs phases de charge consécutives.

Selon un mode de réalisation avantageux, ladite moyenne est calculée en tenant compte des valeurs de la tension **V** mesurée pour six phases de charge consécutives.

Selon des modes de réalisation différents, l'étape **110** de mesure est réalisée à tension **V** constante, en mesurant l'intensité **i,** à tension **V** ou intensité i variables, en mesurant respectivement l'intensité **i** ou la tension **V** ou encore en mesurant à la fois la tension **V** et l'intensité **i.**

L'étape **110** de mesure peut également être réalisée en mesurant une pression ou toute autre grandeur fournie par les capteurs **3**.

Avantageusement, dans une étape **115** d'arrêt du procédé **100** illustré sur la figure 2, l'élément **10** de contrôle suspend les prises de mesure réalisées par l'élément **9** de mesure si la tension **V** mesurée, c'est-à-dire la puissance mesurée, chute brutalement.

La longueur **L** active de chaque capteur **3** est obtenue par la formule suivante, réalisée dans une deuxième étape **122** de calcul du procédé **100** :

$$L = C - C_V \times struct$$

la variable **struct** étant un coefficient, dépendant de la structure du dispositif **1**. Ce coefficient est déterminé expérimentalement. Selon le mode de réalisation, ledit coefficient est égal à 13,5 millimètres par picofarad.

L'aire **A_n** d'un des trapèzes **7** est égale, selon une troisième étape **123** de calcul du procédé **100,** à :

$$A_n = d \times \frac{L_n + L_{n+1}}{2}$$

la variable **L_n** étant la longueur **L** active d'un des capteurs **3** numéroté **n** et **L_{n+1}** étant la longueur **L** active d'un capteur **3** numéroté **n+1** voisin dudit capteur **3** numéroté **n.**

La surface **S** de contact du corps de l'individu avec la protection est donc égale, selon une quatrième étape **124** de calcul du procédé **100** pour un dispositif **1** comprenant dix-huit capteurs **3**, à :

$$S = \sum_{n=1}^{18} A_n S = \sum_{n=1}^{18} d \times \frac{L_n + L_{n+1}}{2}$$

Le calcul de la surface **S** de contact par l'élément **10** de contrôle à la quatrième étape **124** de calcul du procédé **100** permet de vérifier la présence d'un individu sur le lit, et d'évaluer le niveau de mouvement de l'individu.

Dans une mise en œuvre, la présence d'un individu sur le lit s'exprime, à une étape **130** de détection de présence d'un individu du procédé **100,** par la résolution de l'inéquation suivante, pendant une durée supérieure à 800 millisecondes :

$$S > R \times \frac{20}{100}$$

Afin d'adapter la sensibilité de la détection à tout individu adulte, la surface **R** de référence est choisie, selon un mode de réalisation, à une valeur correspondant à un individu dont la masse est de 45 kilogrammes. A titre d'exemple, la surface **R_{min}** de référence minimale, pour un individu dont la masse est de 45 kilogrammes, est de 155000 millimètres carrés.

Selon des modes de réalisation différents, la surface **R_{min}** de référence minimale est choisie pour un individu dont la masse est différente. Avantageusement, la surface **R_{min}** de référence minimale est choisie en fonction des caractéristiques directes de l'individu reposant sur le lit.

Selon un mode de réalisation, la présence d'un individu sur le lit s'exprime, à l'étape **130** de détection de présence d'un individu, par la résolution de l'inéquation suivante, pendant une durée supérieure à 800 millisecondes :

$$S > R_{min} \times \frac{20}{100}$$

soit

$$S > 155000 \times \frac{20}{100}$$

soit

$$S > 31000$$

Si la présence d'un individu est détectée à l'étape **130** de détection de présence, une étape **140** de détection d'absence de mouvement est réalisée par l'élément **10** de contrôle.

L'absence de mouvement d'un individu présent sur le lit s'exprime, pour des capteurs **3** ayant une surface de 3444 millimètres carrés, par la résolution de l'inéquation suivante :

$$\Delta S < 3 \times \frac{3444}{400/1000}$$

soit

$$\Delta S < 25830$$

la variable $\Delta$**S(t)** étant la variation $\Delta$**S** de la surface **S** de contact du corps de l'individu avec le lit pendant une durée de 400 millisecondes.

Afin de vérifier cette inégalité, l'élément **10** de contrôle réalise une cinquième étape **125** de calcul en amont de l'étape **140** de détection d'absence de mouvement.

Cette cinquième étape **125** de calcul permet de calculer la valeur $\Delta$**S(t)** de la variation $\Delta$**S** de la surface **S** de contact du corps de l'individu avec le lit pendant une durée de 400 millisecondes.

Une variation $\Delta$**A_n** de l'aire d'un des trapèzes **7** en fonction du temps, est exprimée par l'équation suivante :

$$\Delta A_n(t) = \frac{|A_n(t) - A_n(t - dt)|}{dt}$$

La variable **A_n(t)** étant l'aire **A_n** d'un des trapèzes **7** à l'instant **t** et dt étant la période de temps séparant deux mesures.

Ainsi :

$$\Delta A_n(t) = d \times \frac{|L_n(t) - L_n(t - dt) + L_{n+1}(t) - L_{n+1}(t - dt)|}{2dt}$$

Cette équation nous permet de calculer la valeur $\Delta$**S(t)** de la variation $\Delta$**S** de la surface **S** de contact du corps de l'individu avec le lit pendant la durée séparant deux mesures, soit, dans le cas présent, 400 millisecondes :

$$\Delta S(t) = \sum_{n=1}^{18} \Delta A_n(t) \Delta S(t) = \frac{d}{2dt} \sum_{n=1}^{18} |L_n(t) - L_n(t - dt) + L_{n+1}(t) - L_{n+1}(t - dt)|$$

Selon un mode de réalisation différent, la cinquième étape **125** de calcul est réalisée à un autre moment du procédé **100**, tant que cette cinquième étape **125** de calcul est réalisée après la quatrième étape **124** de calcul et avant l'étape **140** de détection d'absence de mouvement.

Si l'absence de mouvement n'est pas détectée à l'étape **140** de détection d'absence de mouvement, une étape **150** de détection d'amplitude des mouvements est réalisée par l'élément **10** de contrôle.

Le niveau d'amplitude des mouvements d'un individu présent sur le lit s'exprime par la résolution de l'inéquation suivante, pendant une durée de 400 millisecondes :

$$\Delta S > R \times \frac{11}{100}$$

Si l'inéquation est vérifiée, alors l'individu reposant sur le lit effectue de grands mouvements. Sinon, celui-ci effectue de petits mouvements.

Afin d'adapter la sensibilité de la détection à un individu moyen, la surface **R** de référence est choisie, selon le mode de réalisation préférée, comme surface $\mathbf{R}_{moy}$ de référence moyenne.

A titre d'exemple, la surface $\mathbf{R}_{moy}$ de référence moyenne, pour un individu moyen, est de 350000 millimètres carrés.

Selon des modes de réalisation différents, la surface $\mathbf{R}_{moy}$ de référence moyenne est différente. Avantageusement, la surface $\mathbf{R}_{moy}$ de référence moyenne est choisie en fonction des caractéristiques directes de l'individu reposant sur le lit.

Selon un mode de réalisation, l'amplitude des mouvements d'un individu présent sur le lit s'exprime donc, à l'étape 150 de détection d'amplitude des mouvements, par la résolution de l'inéquation suivante :

$$\Delta S(t) > R_{moy} \times \frac{11}{100} \times \frac{1}{400/1000}$$

soit

$$\Delta S(t) > 350000 \times \frac{11}{100} \times \frac{1}{400/1000}$$

soit

$$\Delta S(t) > 96250$$

Selon un mode de réalisation avantageux, l'élément **10** de contrôle est apte à émettre, dans une étape **160** de notification du procédé **100,** une ou plusieurs alertes ou informations tenant compte des résultats de l'étape **130** de détection de présence d'un individu, de l'étape **140** de détection d'absence de mouvement et de l'étape **150** de détection d'amplitude des mouvements.

Ainsi, l'étape **160** de notification comprend une étape **161** d'information émettant les résultats des étapes **130, 140, 150** de détection ci-dessus.

L'étape **161** d'information est émise régulièrement, de sorte à permettre le suivi de l'état de l'individu reposant sur le lit.

Selon un mode de réalisation différent, l'étape **161** d'information est émise à la demande d'une personne souhaitant connaître l'état dudit individu.

L'étape **161** d'information renseigne sur l'état actuel de l'individu et/ou sur son état passé, par exemple pendant les dernières 24 heures.

Les résultats des étapes **130, 140, 150** de détection ci-dessus sont également émis sous formes d'alertes, visant la personne suivant l'état de l'individu reposant sur le lit.

Selon un mode de réalisation, l'étape **160** de notification comprend une étape **162** d'alerte de non-présence de l'individu, avertissant ladite personne si l'individu ne repose pas sur le lit pendant un temps prédéterminé.

Si l'individu est autonome, l'étape **162** d'alerte de non-présence de l'individu survient, par exemple, lorsqu'une absence du lit est détectée, à l'étape **130** de détection de présence d'un individu, entre 8 heures du soir et 8 heure du matin pendant une durée supérieure à 15 minutes, laissant ainsi le temps à l'individu d'aller, par exemple, aux toilettes.

Si l'individu n'est pas autonome, l'étape **162** d'alerte de non-présence de l'individu survient, par exemple, lorsqu'une absence du lit est détectée, à l'étape **130** de détection de présence d'un individu, pendant plus de 5 secondes.

L'étape **160** de notification comprend également une étape **163** d'alerte d'absence de mouvement, si une absence de mouvement de l'individu est détectée à l'étape **140** de détection d'absence de mouvement.

L'étape **160** de notification comprend une étape **164** d'alerte d'amplitude des mouvements, si l'étape **150** de détection d'amplitude des mouvements révèle que l'individu réalise des petits mouvements pendant un temps prédéterminé.

Selon un mode de réalisation différent, l'étape **160** de notification comprend également une étape d'alerte d'agitation, si l'étape **140** de détection d'absence de mouvement révèle que l'individu réalise un nombre prédéterminé de mouvements pendant un temps donné.

Selon d'autres modes de réalisation, l'étape d'alerte d'agitation est réalisée si l'étape **150** de détection d'amplitude des mouvements révèle que l'individu réalise un nombre prédéterminé de grands ou de petits mouvements pendant un temps donné.

Selon le mode de réalisation préféré, les émissions de l'étape **161** d'information et des étapes **162, 163, 164** d'alerte ci-dessus sont réalisées sur un terminal **11** de suivi.

Le terminal **11** de suivi est avantageusement relié au boîtier **4** par l'intermédiaire d'un réseau sans fil.

Le terminal **11** de suivi est fixe et/ou mobile.

Selon des modes de réalisation différents, l'émission est réalisée par le biais d'un réseau filaire et/ou le terminal **11** de suivi comprend un écran apte à afficher l'étape **161** d'information et les étapes **162, 163, 164** d'alerte.

Le boîtier **4** comprend, de préférence, une mémoire **12** apte à mémoriser des paramètres permettant une identification de l'individu, de ses paramètres physiques et/ou de paramètres par défaut, permettant de réaliser les différentes étapes du procédé **100.**

La mémoire **12** est également apte à mémoriser les résultats des étapes **130, 140, 150** de détection.

Selon un mode de réalisation différent, les étapes **121, 122, 123, 124,125** de calcul ne calculent pas des grandeurs physiques mais des indicateurs de ces grandeurs.

Ainsi, la première étape **121** de calcul calcule un indicateur de la capacité **C,** la deuxième étape **122** de calcul calcule un indicateur de la longueur **L,** la troisième étape **123** de calcul calcule un indicateur de l'aire $A_n$, la quatrième étape **124** de calcul calcule un indicateur de la surface **S** et la cinquième étape **125** de calcul calcule une variation $\Delta S$ de l'indicateur de la surface **S.** Les étapes **130, 140, 150** utilisent ces indicateurs.

Le procédé et le dispositif selon l'invention présentent de nombreux avantages.

Les informations fournies par la nappe de capteurs permettent de fournir un indicateur de risque d'escarres, cet indicateur ne présentant pas de subjectivité liée à un observateur, l'indicateur résultant d'une observation automatique, longue et continue des mouvements de la personne.

En fonction de la valeur de cet indicateur, une alerte est générée et communiquée par exemple au personnel soignant.

Les informations fournies par la nappe de capteur permettent en outre d'alerter le personnel soignant de risques de chute, de déambulation, ou d'agitation d'une personne.

Les informations fournies par la nappe de capteur peuvent être accessibles sur terminal de communication fixe ou mobile, notamment en mode push pour les alertes. Avantageusement, un système d'acquittement assure qu'une information diffusée en mode push a été vue par son destinataire (par exemple un membre de la famille ou un personnel soignant), une alerte étant générée en cas d'absence d'acquittement.

La nappe de capteurs se présente avantageusement sous la forme d'une housse pour matelas de dimension standard. La mise en place de la nappe de capteur est ainsi facilitée. Avantageusement, la housse est lavable, l'invention n'ayant pas d'impact sur l'hygiène de la personne alitée. Avantageusement, la nappe de capteur est disposée entre un matelas et une housse amovible de protection, notamment housse en textile enduit.

La présence de la nappe de capteurs et les moyens de traitement de l'information fournie par les capteurs sont sans impact notable sur le confort du patient.

L'efficacité de la méthode dans la prévention des escarres peut être facilement démontrée, notamment par comparaison avec un groupe témoin.

Les données numériques fournies dans la description ci-dessus sont appliquées au mode de réalisation décrit et ne sont en aucun cas limitatives.

| Faisceau comprenant | | | Capteurs situés du côté de la tête | | | | | | | | | | | | | | Capteurs situés du côté des pieds | | | |
|---|---|---|---|---|---|---|---|---|---|---|---|---|---|---|---|---|---|---|---|---|---|
| | 1 partie | Longueur du faisceau entre chaque capteur et le boîtier (mm) | 850 | 750 | 650 | 550 | 450 | 350 | 250 | 150 | 50 | 50 | 150 | 250 | 350 | 450 | 550 | 650 | 750 | 850 |
| | | Capacité générée par le faisceau entre chaque capteur et le boîtier (pF) | 41,9 | 38,0 | 34,0 | 30,1 | 26,1 | 22,2 | 18,3 | 14,3 | 10,4 | 10,4 | 14,3 | 18,3 | 22,2 | 26,1 | 30,1 | 34,0 | 38,0 | 41,9 |
| | | Capacité moyenne générée par le faisceau entre les capteurs et le boîtier (pF) | 26,1 | | | | | | | | | | | | | | | | | |
| | 2 parties | Longueur du faisceau entre chaque capteur et le boîtier (mm) | 400 | 300 | 200 | 100 | 0 | 100 | 200 | 300 | 400 | 400 | 300 | 200 | 100 | 0 | 100 | 200 | 300 | 400 |
| | | Capacité générée par le faisceau entre chaque capteur et le boîtier (pF) | 24,2 | 20,2 | 16,3 | 12,4 | 8,4 | 12,4 | 16,3 | 20,2 | 24,2 | 24,2 | 20,2 | 16,3 | 12,4 | 8,4 | 12,4 | 16,3 | 20,2 | 24,2 |
| | | Capacité moyenne générée par le faisceau entre les capteurs et le boîtier (pF) | 17,2 | | | | | | | | | | | | | | | | | |
| | 3 parties | Longueur du faisceau entre chaque capteur et le boîtier (mm) | 250 | 150 | 50 | 50 | 150 | 250 | 250 | 150 | 50 | 50 | 150 | 250 | 250 | 150 | 50 | 50 | 150 | 250 |
| | | Capacité générée par le faisceau entre chaque capteur et le boîtier (pF) | 18,3 | 14,3 | 10,4 | 10,4 | 14,3 | 18,3 | 18,3 | 14,3 | 10,4 | 10,4 | 14,3 | 18,3 | 18,3 | 14,3 | 10,4 | 10,4 | 14,3 | 18,3 |
| | | Capacité moyenne générée par le faisceau entre les capteurs et le boîtier (pF) | 14,3 | | | | | | | | | | | | | | | | | |

Tableau T

**Revendications**

1. Procédé **(100)** pour fournir un indicateur de risques d'escarres d'un individu reposant sur un support, le procédé **(100)** comprenant :

   - une étape **(110)** de mesure d'au moins une grandeur, par le biais de capteurs **(3)** disposés entre le support et l'individu ;
   - une étape **(121, 122, 123, 124, 125)** de calcul d'un indicateur d'une surface **(S)** de contact de l'individu avec le support, à partir de ladite au moins une grandeur ;
   - une étape **(130)** de détection de présence de l'individu sur le support, en fonction de l'indicateur de la surface **(S)** de contact de l'individu avec le support ;
   - une étape **(140)** de détection d'absence de mouvement réalisé par l'individu, en fonction d'une variation ($\triangle$**S**) dans le temps de l'indicateur de la surface **(S)** de contact calculée, si, au cours de l'étape **(130)** de détection de présence, la présence d'un individu est détectée sur le support ;
   - une étape **(150)** de détection d'amplitude des mouvements réalisés par l'individu, en fonction de la variation ($\triangle$**S**) dans le temps de l'indicateur de la surface **(S)** de contact calculée, si, au cours de l'étape **(140)** de détection d'absence de mouvement, une absence de mouvement n'est pas détectée ;
   - une étape **(160)** de notification de l'état de l'individu.

2. Procédé selon la revendication précédente, **caractérisé en ce que** l'étape **(160)** de notification de l'état de l'individu comprend :

   - une étape **(161)** d'information de l'état de l'individu émettant des résultats de l'étape **(130)** de détection de présence de l'individu, de l'étape **(140)** de détection d'absence de mouvement et de l'étape **(150)** de détection d'amplitude des mouvements, cette étape **(161)** d'information étant émise régulièrement ;
   - une étape **(162)** d'alerte de non-présence de l'individu sur le support, cette étape **(162)** d'alerte de non-présence de l'individu étant émise si, à l'étape **(130)** de détection de présence de l'individu, aucun individu n'est détecté pendant un temps prédéterminé ;
   - une étape **(163)** d'alerte d'absence de mouvement de l'individu, cette étape **(163)** d'alerte d'absence de mouvement étant émise si, à l'étape **(140)** de détection d'absence de mouvement, aucun mouvement n'est détecté pendant un temps prédéterminé ;
   - une étape **(164)** d'alerte d'amplitude des mouvements de l'individu, cette étape **(164)** d'alerte d'amplitude des mouvements étant émise si, à l'étape **(150)** de détection d'amplitude des mouvements, un petit mouvement est détecté pendant un temps prédéterminé.

3. Procédé selon l'une quelconque des revendications précédentes, **caractérisé en ce que** l'étape **(160)** de notification comprend une étape d'alerte d'agitation de l'individu, cette étape d'alerte d'agitation étant fonction d'un nombre prédéterminé de mouvements détectés pendant un temps donné.

4. Procédé selon l'une quelconque des revendications précédentes, **caractérisé en ce que** ladite au moins une grandeur mesurée à l'étape **(110)** de mesure est une grandeur électrique et l'étape **(121, 122, 123, 124, 125)** de calcul comprend:

   - une première étape **(121)** de calcul d'un indicateur d'une capacité **(C)** générée par les capteurs **(3)** ;
   - une deuxième étape **(122)** de calcul d'un indicateur d'une longueur **(L)** active de chaque capteur **(3)** ;
   - une troisième étape **(123)** de calcul d'un indicateur d'une aire **($A_n$)** ;
   - une quatrième étape **(124)** de calcul de l'indicateur de la surface **(S)** de contact ;
   - une cinquième étape **(125)** de calcul de la variation ($\triangle$**S**) dans le temps de l'indicateur de la surface **(S)** de contact.

5. Dispositif **(1)** pour la prévention de la formation d'escarres, ce dispositif **(1)** étant apte à réaliser le procédé **(100)** selon l'une quelconque des revendications précédentes, le dispositif **(1)** comprenant :

   - les capteurs **(3)** disposés entre le support et l'individu, les capteurs **(3)** étant disposés de sorte à indiquer la présence d'un objet sur le support ;
   - un boîtier **(4)** relié aux capteurs **(3),** le boîtier **(4)** étant muni :

     ∘ d'un élément **(10)** de contrôle, configuré pour réaliser les étapes du procédé **(100)** ;

◦ d'une mémoire **(12),** apte à enregistrer les étapes du procédé **(100)** ;

- un terminal **(11)** de suivi, apte à afficher l'étape **(160)** de notification de l'état de l'individu.

**6.** Dispositif selon la revendication précédente, **caractérisé en ce que** les capteurs **(3)** présentent chacun la forme d'une bande.

**7.** Dispositif selon l'une quelconque des revendications 5 et 6, **caractérisé en ce que** les capteurs **(3)** sont sensiblement perpendiculaires à une direction suivant laquelle s'étend un corps de l'individu.

**8.** Dispositif selon l'une quelconque des revendications 5 à 7, **caractérisé en ce que** les capteurs **(3)** sont des capteurs **(3)** capacitifs générant la capacité **(C).**

**9.** Dispositif selon l'une quelconque des revendications 5 à 8, **caractérisé en ce qu'**il comprend un faisceau **(5)** reliant les capteurs **(3)** et le boîtier **(4),** le faisceau **(5)** comprenant plusieurs parties **(8)** indépendantes.

**10.** Dispositif selon la revendication précédente, **caractérisé en ce que** le faisceau **(5)** comprend deux parties **(8).**

**Patentansprüche**

**1.** Verfahren **(100)** zur Bereitstellung eines Indikators für Dekubitusrisiken eines Individuums, das auf einer Unterlage ruht, wobei das Verfahren **(100)** umfasst:

- einen Messschritt **(110)** mindestens einer Größe anhand von Sensoren **(3),** die zwischen der Unterlage und dem Individuum angeordnet sind;
- einen Berechnungsschritt **(121, 122, 123, 124, 125)** eines Indikators einer Kontaktfläche **(S)** des Individuums mit der Unterlage auf der Basis der mindestens einen Größe;
- einen Ermittlungsschritt **(130)** der Anwesenheit des Individuums auf der Unterlage in Abhängigkeit von dem Indikator der Kontaktfläche **(S)** des Individuums mit der Unterlage;
- einen Ermittlungsschritt **(140)** der Abwesenheit von Bewegung, durchgeführt von dem Individuum, in Abhängigkeit von einer zeitlichen Variation ($\Delta$**S)** des Indikators der berechneten Kontaktfläche **(S),** wenn während des Ermittlungsschritts **(130)** von Anwesenheit die Anwesenheit eines Individuums auf der Unterlage ermittelt wird;
- einen Ermittlungsschritt **(150)** einer Amplitude der von dem Individuum durchgeführten Bewegungen in Abhängigkeit von der zeitlichen Variation **(AS)** des Indikators der berechneten Kontaktfläche **(S),** wenn während des Ermittlungsschritts **(140)** von Bewegungsabwesenheit keine Bewegungsabwesenheit ermittelt wird;
- einen Informationsschritt **(160)** über den Zustand des Individuums.

**2.** Verfahren nach vorangehendem Anspruch, **dadurch gekennzeichnet, dass** der Informationsschritt **(160)** über den Zustand des Individuums umfasst:

- einen Informationsschritt **(161)** über den Zustand des Individuums, bei dem Ergebnisse des Ermittlungsschritts **(130)** von Anwesenheit des Individuums, des Ermittlungsschritts **(140)** von Bewegungsabwesenheit und des Ermittlungsschritts **(150)** von Bewegungsamplitude ausgegeben werden, wobei dieser Informationsschritt **(161)** regelmäßig erfolgt;
- einen Warnschritt **(162)** bezüglich Nicht-Anwesenheit des Individuums auf der Unterlage, wobei dieser Warnschritt **(162)** bezüglich Nicht-Anwesenheit des Individuums erfolgt, wenn beim Ermittlungsschritt **(130)** von Anwesenheit des Individuums kein Individuum während einer vorher festgelegten Zeit ermittelt wird;
- einen Warnschritt **(163)** bezüglich Bewegungsabwesenheit des Individuums, wobei dieser Warnschritt **(163)** bezüglich Bewegungsabwesenheit erfolgt wird, wenn beim Ermittlungsschritt **(140)** von Bewegungsabwesenheit keine Bewegung während einer vorher festgelegten Zeit ermittelt wird;
- einen Warnschritt **(164)** bezüglich der Amplitude der Bewegungen des Individuums, wobei dieser Warnschritt **(164)** bezüglich der Amplitude der Bewegungen erfolgt, wenn beim Ermittlungsschritt **(150)** einer Bewegungsamplitude eine kleine Bewegung während einer vorher festgelegten Zeit ermittelt wird.

**3.** Verfahren nach einem der vorangehenden Ansprüche, **dadurch gekennzeichnet, dass** der Informationsschritt **(160)** einen Warnschritt bezüglich Unruhe des Individuums umfasst, wobei dieser Warnschritt bezüglich Unruhe von einer vorher festgelegten Anzahl von Bewegungen abhängt, die in einer bestimmten Zeit ermittelt werden.

4.  Verfahren nach einem der vorangehenden Ansprüche, **dadurch gekennzeichnet, dass** die mindestens eine beim Messschritt **(110)** gemessene Größe eine elektrische Größe ist und dass der Berechnungsschritt **(121, 122, 123, 124, 125)** umfasst:

    - einen ersten Berechnungsschritt **(121)** eines Indikators einer von den Sensoren **(3)** erzeugten Kapazität **(C);**
    - einen zweiten Berechnungsschritt **(122)** eines Indikators einer aktiven Länge **(L)** jedes Sensors **(3);**
    - einen dritten Berechnungsschritt **(123)** eines Indikators eines Bereichs **($A_n$);**
    - einen vierten Berechnungsschritt **(124)** des Indikators der Kontaktfläche **(S);**
    - einen fünften Berechnungsschritt **(125)** der zeitlichen Variation **($\Delta S$)** des Indikators der Kontaktfläche **(S).**

5.  Vorrichtung **(1)** zur Vorbeugung von Dekubitusbildung, wobei diese Vorrichtung **(1)** imstande ist, das Verfahren **(100)** nach einem der vorangehenden Ansprüche durchzuführen, wobei die Vorrichtung **(1)** umfasst:

    - die Sensoren **(3),** die zwischen der Unterlage und dem Individuum angeordnet sind, wobei die Sensoren **(3)** derart angeordnet sind, dass die Anwesenheit eines Objekts auf der Unterlage angezeigt wird;
    - ein Gehäuse **(4),** das mit den Sensoren **(3)** verbunden ist, wobei das Gehäuse **(4)** versehen ist mit:

        ◦ einem Steuerelement **(10),** das ausgelegt ist, um die Schritte des Verfahrens **(100)** durchzuführen;
        ◦ einem Speicher **(12),** der imstande ist, die Schritte des Verfahrens **(100)** zu speichern;

    - ein Überwachungsendgerät **(11),** das imstande ist, den Informationsschritt **(160)** über den Zustand des Individuums anzuzeigen.

6.  Vorrichtung nach vorangehendem Anspruch, **dadurch gekennzeichnet, dass** die Sensoren **(3)** jeweils die Form eines Bandes aufweisen.

7.  Vorrichtung nach einem der Ansprüche 5 und 6, **dadurch gekennzeichnet, dass** die Sensoren **(3)** etwa senkrecht zu einer Richtung sind, in der sich ein Körper des Individuums erstreckt.

8.  Vorrichtung nach einem der Ansprüche 5 bis 7, **dadurch gekennzeichnet, dass** die Sensoren **(3)** kapazitive Sensoren **(3)** sind, die die Kapazität **(C)** erzeugen.

9.  Vorrichtung nach einem der Ansprüche 5 bis 8, **dadurch gekennzeichnet, dass** sie einen Strahl **(5)** umfasst, der die Sensoren **(3)** und das Gehäuse **(4)** verbindet, wobei der Strahl **(5)** mehrere unabhängige Teile **(8)** umfasst.

10. Vorrichtung nach vorangehendem Anspruch, **dadurch gekennzeichnet, dass** der Strahl **(5)** zwei Teile **(8)** umfasst.

**Claims**

1.  A method (100) for providing an indicator of risks of pressure ulcers for an individual lying on a support, the method (100) comprising:

    - a step (110) of measuring at least one quantity, through sensors (3) disposed between the support and the individual;
    - a step (121, 122, 123, 124, 125) of calculating an indicator of a contact surface (S) of the individual with the support, from said at least one quantity;
    - a step (130) of detecting the presence of the individual on the support, as a function of the indicator of the contact surface (S) of the individual with the support;
    - a step (140) of detecting the absence of movement made by the individual, as a function of a variation ($\Delta S$) over time of the indicator of the calculated contact surface (S), if, during the presence detecting step (130), the presence of an individual is detected on the support;
    - a step (150) of detecting the amplitude of movements made by the individual, as a function of the variation ($\Delta S$) over time of the indicator of the calculated contact surface (S), if, during the step (140) of detecting the absence of movement, an absence of movement is not detected;
    - a step (160) of notifying the condition of the individual.

2.  The method according to the previous claim, **characterised in that** the step (160) of notifying the condition of the individual comprises:

- a step (161) of informing about the condition of the individual transmitting results of the step (130) of detecting the presence of the individual, of the step (140) of detecting the absence of movement and the step (150) of detecting the amplitude of movements, this informing step (161) being regularly transmitted;
- a step (162) of alerting about the non-presence of the individual on the support, this step (162) of alerting about the non-presence of the individual being transmitted if, in the step (130) of detecting the presence of the individual, no individual is detected for a predetermined time;
- a step (163) of alerting about the absence of movement of the individual, this step (163) of alerting about the absence of movement being transmitted if, in the step (140) of detecting the absence of movement, no movement is detected for a predetermined time;
- a step (164) of alerting about the amplitude of movements of the individual, this step (164) of alerting about the amplitude of movements being transmitted if, in the step (150) of detecting the amplitude of movements, a small movement is detected for a predetermined time.

3. The method according to any of the previous claims, **characterised in that** the notifying step (160) comprises a step of alerting about agitation of the individual, this agitation alerting step being a function of a predetermined number of movements detected for a given time.

4. The method according to any of the previous claims, **characterised in that** said at least one quantity measured in the measuring step (110) is an electric quantity and the calculating step (121, 122, 123, 124, 125) comprises:

- a first step (121) of calculating an indicator of a capacitance (C) generated by the sensors (3);
- a second step (122) of calculating an indicator of an active length (L) of each sensor (3);
- a third step (123) of calculating an indicator of an area ($A_n$);
- a fourth step (124) of calculating the indicator of the contact surface (S);
- a fifth step (125) of calculating the variation ($\Delta S$) over time of the indicator of the contact surface (S).

5. A device (1) for preventing pressure ulcer formation, this device (1) being able to carry out the method (100) according to any of the previous claims, the device (1) comprising:

- the sensors (3) disposed between the support and the individual, the sensors (3) being disposed so as to indicate the presence of an object on the support;
- a package (4) connected to the sensors (3), the package (4) being provided with:

  ○ a control element (10), configured to carry out the steps of the method (100);
  ○ a memory (12), able to record the steps of the method (100);

- a tracking terminal (11), able to display the step (160) of notifying the condition of the individual.

6. The device according to the previous claim, **characterised in that** the sensors (3) each have the shape of a strip.

7. The device according to any of claims 5 and 6, **characterised in that** the sensors (3) are substantially perpendicular to a direction along which the individual's body extends.

8. The device according to any of claims 5 to 7, **characterised in that** the sensors (3) are capacitive sensors (3) generating the capacitance (C).

9. The device according to any of claims 5 to 8, **characterised in that** it comprises a beam (5) connecting the sensors (3) and the package (4), the beam (5) comprising several independent parts (8).

10. The device according to the previous claim, **characterised in that** the beam (5) comprises two parts (8).

# Fig. 1

Fig. 2

Fig. 3

Fig. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1664712 A
- JP H11342161 B, Denso
- JP H8238275 B

- EP 2508128 A
- US 2005172398 A

**Littérature non-brevet citée dans la description**

- **COLEMAN et al.** Patient risk factors for pressure ulcer development : systematic review. *International journal of nursing studies,* 2013, 974-1003
- **ANTHONY et al.** Norton, Waterlow and Braden scores : a review of the literature and a comparison between the scores and clinical judgment. *Journal of Clinical Nursing,* 2008, 646-653
- **CHEN et al.** Braden scale for assessing ulcer risk in hospital patients : a validity and reliability study. *Applied Nursing Research,* 2017, 169-174
- **SERPA et al.** Validity of the Braden and Waterloo subscales in predicting pressure ulcer risk in hospitalized patients. *Applied Nursing Research,* 2011, e23-e28
- **SEONGSOOK et al.** Validity of pressure ulcer risk assessment, Cubbin and Jackson, Braden and Douglas scale. *International Journal of Nursing Studies,* 2004, 199-204
- **BOISSON.** Optimisation de la prévention ambulatoire des ulcères cutanés dus à la pression. *Thèse de docteur en pharmacie,* 2016

- **TORRESSAN.** Prévalence des escarres dans les établissements pour personnes âgées dépendantes en Aquitaine. *Thèse,* 2015
- **DÉMARRÉ et al.** The cost of prévention and treatment of pressure ulcers : a systematic review. *International journal of nursing studies,* 2015, 1754-1774
- **MEFFRE et al.** Mapi : active interface pressure sensor intergrated into a seat. *IRBM,* 2008, 375-379
- **MEFFRE.** Conception et réalisation d'une instrumentation dédiée à la prédiction du confort d'assise et à la prévention des escarres. *Thèse INSA Lyon,* 2007
- **MARCHIONE et al.** Approaches that use software to support the prevention of pressure ulcer : as systematic review. *International Journal of Medical Informatics,* 2015, 725-736
- **PANFIL et al.** Häufigkeit von Bewegung im Bett zur Einschätzung der Dekubitusgefahrdung - Eine systematisierte Übersichtsarbeit. *International journal of health professions,* 2014, 61-72